# EUROPEAN PATENT APPLICATION

(11) **EP 2 550 953 A1**
(43) Date of publication of application: **30.01.2013**
(21) Application number: 11175639.1
(22) Date of filing: 27.07.2011
(51) Int. Cl.: A61K 8/04, A61K 8/31, A61K 8/34, A61K 8/81, A61K 8/86, A61Q 5/06, A61K 8/42

(54) **Post-foaming gel composition**

(71) Applicant: KPSS-Kao Professional Salon Services GmbH, 64297 Darmstadt (DE)
(72) Inventor: Sulzbach, Melina, 61440 Oberursel (DE); Weichaus, Dirk, 64404 Bickenbach (DE)

(57) **Abstract**

Present invention relates to a post foaming gel composition for hair for improving its shine and curl retention, reducing flyaways, and setting. Post-foaming aqueous gel composition of the present invention comprises one or more thickening polymer, one or more film forming polymer, one or more polyol, one or more surfactant and one or more C4 - C5 isoalkane and it is easily and homogeneously applied onto hair after releasing from its packaging and it improves shine and curl retention of hair, reduces flyaways and has moderately good setting effect on hair.

## Description

Present invention relates to a post foaming gel composition for hair for improving its shine and curl retention, reducing flyaways, and setting.

Gel type of hair styling compositions have been know for sometime and usually used for setting hair into a certain desired shape. Gel types of compositions are usually packed into a jar although there are gels packed in a tube have also been made available for users. Their application onto hair is usually carried out taking certain parts from the jar and first distributing it on hand surface which is followed by transferring the product from hand to hair surface. This way of application is very ineffective and brings largely microbiological contamination which limits the product life. Furthermore, such products are not homogeneously distributed onto hair surface so that hair style retention is also not very optimal all over the whole head.

Foam applications have been know to overcome such difficulties which required usually high concentrations of surfactants and additionally the gelling ingredients blocks release of the compositions from the packaging because of their high viscosity.

Present invention starts from the above mentioned problems and provides solution to the one or more of them.

The inventors of the present invention has found out that a gel composition forming a foam after its release from its packaging is easily applied onto hair homogeneously and hair styled with such composition is put in the desired from homogeneously and the purchased product may be used entirely as no contamination problem exists.

A post foaming gel composition is known from EP 876 204 B1 which comprises high level of surfactants and therefore is not favorable as a finish product and especially on wet hair.

Accordingly, present invention is on a post-foaming aqueous gel composition comprising one or more thickening polymer, one or more film forming polymer, one or more polyol, one or more surfactant and one or more C4 - C5 isoalkane which is easily and homogeneously applied onto hair after releasing from its packaging and it improves shine and curl retention of hair, reduces flyaways and has moderately good setting effect on hair.

The first object of the present invention is a post-foaming aqueous gel composition comprising one or more thickening polymer, one or more film forming polymer, one or more polyol, one or more surfactant and one or more C4 - C5 isoalkane.

Further object of the present invention is the use of the post foaming aqueous gel composition for treating hair.

Further object of the present invention is process for treating hair wherein the post foaming aqueous gel composition is applied onto hair, preferably onto wet hair.

Another object of the present invention is a pressurized product for treating hair which comprises a pressure resistant vessel consisting of two chambers wherein one of the chambers comprises the post foaming aqueous gel composition and the other chamber comprises at least one pressurizing gas wherein the pressure inside the vessel does not exceed 12 bar. In a preferred embodiment of the present invention the two chambers of the pressure resistant vessel are separated from each other with a movable piston.

Aqueous post-foaming gel compositions of the present invention comprise one or more thickening polymer. In principal any polymer having a thickening effect in an aqueous environment is suitable for the purpose of the present invention. In a preferred embodiment, the thickening polymers are selected from the ones forming a thixotropic gel in an aqueous medium. Suitable ones are nonionic polymers such as xanthan gum, cellulosic polymers and their derivatives and anionic polymers such as acrylate type of polymers. Most preferred are alkyl acrylate types of polymers such as acrylates / palmeth-25 acrylate copolymer.

Concentration of one or more thickening polymer is in the range of 0.1 to 5%, preferably 0.1 to 4%, more preferably 0.2 to 3% and most preferably 0.25 to 2% by weight, calculated to the total composition.

Compositions of the present invention comprise one or more film forming polymers. In principal any film forming polymer known is suitable for the purpose of the present invention. Preferably the polymers are selected from the nonionic, anionic, cationic and amphoteric ones.

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG and advantage LS-E from ISP.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl - methacrylates of the type "Yukaformer®", e.g.. the butyl methacrylate copolymer "Yukaformer® Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Suitable anionic polymers alone or in combination with non-ionic polymers are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further suitable anionic polymers are Acrylate copolymers available under trade name Salcare SC 81, PEG/PPG 25/25 dimethicone / acrylate copolymer available under trade name Luviglex Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Suitable cationic polymers are such as Polyquaternium 11, Polyquaternium 16, Polyquaternium 22 and Polyquaternium 28, Polyquaternium 30, Polyquaternium 37, Polyquaternium 36, Polyquaternium 46, Polyquaternium 24, Polyquaternium 67, and Polyquaternium 72.

Concentration of one or more film forming polymer is in the range of 0.1 to 10%, preferably 0.2 to 7.5%, more preferably 0.25 to 5% and most preferably 0.55 to 2.5% by weight, calculated to the total composition.

Compositions of the present invention comprise one or more polyol. With the term polyol it is meant that the compound has 2 or more free hydroxyl group in its molecule. Suitable and preferred ones are such as panthenol, propylene glycol and glycerin and their mixtures. Especially preferred is the mixture of glycerin and propylene glycol.

Concentration of one or more polyol is in the range of 5 to 30%, preferably 7.5 to 25%, more preferably 7.5 to 20% and most preferably 8 to 17% by weight, calculated to the total composition.

The post-foaming gel composition comprises one or more surfactants. Suitable ones are of anionic, non-ionic, amphoteric and cationic surfactants or their mixtures.

As a rule any cationic surfactant is suitable for the compositions of the present invention. Preferably at least one cationic surfactant is selected from the compounds with the general formula 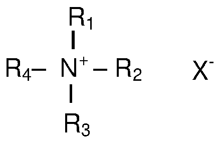 where R₁s a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Non-limiting examples to suitable cationic surfactants are cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, stearamidopropyldimethyl ammonium chloride.

Suitable non-ionic surfactants are alkyl polyglucosides of the general formula

R₇ - O - (R₈O)ₙ O - Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid mono and di ethanolamide and myristic fatty acid mono and di ethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants useful in the compositions according to invention are C₁₀-C₂₂-fatty alcohol ethoxylates. Suitable non-limiting examples are oleth-10, oleth-11, oleth-12, oleth-15, oleth-16, oleth-20, oleth-25, oleth-30, oleth-35, oleth-40, laureth-10, laureth-11, laureth-12, laureth-13, laureth-15, laureth-16, laureth-20, laureth-25, laureth-30, laureth-35, laureth-40, laureth-50, ceteth-10, ceteth-12, ceteth-14, ceteth-15, ceteth-16, ceteth-17, ceteth-20, ceteth-25, ceteth-30, ceteth-40, ceteth-45, cetoleth-10, cetoleth-12, cetoleth-14, cetoleth-15, cetoleth-16, cetoleth-17, cetoleth-20, cetoleth-25, cetoleth-30, cetoleth-40, cetoleth-45, ceteareth-1 0, ceteareth-12, ceteareth-14, ceteareth-15, ceteareth-16, ceteareth-18, ceteareth-20, ceteareth-22, ceteareth-25, ceteareth-30, ceteareth-40, ceteareth-45, ceteareth-50, isosteareth-10, isosteareth-12, isosteareth-15, isosteareth-20, isosteareth-22, isosteareth-25, isosteareth-50, steareth-1 0, steareth-11, steareth-14, steareth-15, steareth-16, steareth-20, steareth-25, steareth-30, steareth-40, steareth-50, steareth-80 and steareth-100. Additional examples of similar compounds can be found in the cosmetic ingredient dictionaries and cosmetic textbooks.

Further non-ionic surfactants within the meaning of the present invention are polyalkyleneglycol ether of fatty acid glyceride or partial glyceride with at least 30 polyalkylene units are with 30 to 1000, preferably 30 to 500, more preferably 30 to 200 and most preferably 40 to 100 polyethyleneglycol units. Examples to those are PEG-30 hydrogenated castor oil, PEG-35 hydrogenated castor oil, PEG-40 hydrogenated castor oil, PEG-45 hydrogenated castor oil, PEG-50 hydrogenated castor oil, PEG-55 hydrogenated castor oil, PEG-60 hydrogenated castor oil, PEG-65 hydrogenated castor oil, PEG-80 hydrogenated castor oil, PEG-100 hydrogenated castor oil, PEG-200 hydrogenated castor oil, PEG-35 castor oil, PEG-50 castor oil, PEG-55 castor oil, PEG-60 castor oil, PEG-80 castor oil, PEG-1 00 castor oil, PEG-200 castor oil. Additional examples of similar compounds can be found in the cosmetic ingredient dictionaries and cosmetic textbooks.

Further suitable non-ionic surfactants are monoglycerides such as glyceryl stearate, glyceryl palmitate, glyceryl myristate, glyceryl behenate.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate.

Further, suitable within the meaning of the present invention are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-, di- or tri alkyl phosphates.

Additional anionic surfactants useful are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉ - (C₂H₄O)ₙ - O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Among the suitable surfactants, preferred surfactants are non-ionic surfactants and among those ethoxylated fatty alcohols and ethoxylated gylcerides and their mixtures are most preferred.

Concentration of one or more surfactant in the compositions according to present invention is not exceeding 5% and preferably in the range of 0.1 to 5%, more preferably 0.2 to 4% and most preferably 0.25 to 3% by weight calculated to total composition.

Compositions of the present invention comprise one or more C4 to C5 isoalkane. Suitable and most preferred are isopentane and isobutene at a concentration of 1 to 20%, preferably in the range of 2 to 15%, more preferably 2 to 12% and most preferably 2.5 to 10% by weight calculated to total composition.

Composition of the present invention is provided in a pressurised two chamber vessel. One of the two chambers comprises the aqueous gel composition and the other chamber comprises one or more propellant. Suitable propellants are lower alkanes such as n-butane, i-butane, propane, butane, liquid nitrogen, pressurised air, corbondioxide, Hydrogen, oxygen and LPG. Pressure in the can is typically less than 12 bar, preferably around 8.5 bar. Concentration of one or more propellant is in the range of 1 to 30%, preferably 2 to 20% more preferably 5 to 15% by weight calculated to total composition.

The compositions according to the invention may also comprise further agents, such as proteins, for example bamboo protein, and protein hydrolyzates and polypeptides, e.g. keratin hydrolyzates, collagen hydrolyzates of the type "Nutrilan^{®}" or elastin hydrolyzates, as well as, in particular vegetable, optionally cationized protein hydrolyzates, for example "Gluadin^{®}".

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total composition . Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis". 4^{th} Ed..

Compositions of the present invention may contain UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysiliocne-15.

The suitable amount of the UV-absorber ranges from about 0.01 % to 1% by weight, calculated to the total composition. Attention should be paid to the stability and solubility especially when using UV filter as salts, e.g. anionic UV filter salts.

In a further embodiment of the present invention, the compositions comprise at least one direct dye for colouring hair. Suitable direct dyes are cationic, anionic, neutral dyes and their mixtures as available commercially from various suppliers and used mainly in semipermanent hair coloration.

Non-limiting examples to cationic dyes are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dye. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.

The above mentioned dyestuffs are also used, especially the anionic ones, for product coloring at reduced concentrations.

Concentration of direct dyes in the compositions of the present invention is within the range of 0.001 to 5%, preferably 0.01 to 3% and more preferably 0.05 to 2%, and most preferably 0.1 to 1% by weight calculated to total composition, calculated to total composition.

The composition of the present invention is preferably provided in a form of kit together with one or more of additional hair cosmetic products. Therefore, further object of the present invention is kit for hair comprising a composition according to the present invention.

Following examples are to illustrate the invention but not to limit it.

### Example 1

| | % by weight |
|---|---|
| Propylene glycol | 5 |
| Glycerine | 5 |
| VP/VA copolymer | 2 |
| Laureth-23 | 2 |
| Synthalen W 2000 | 5 |
| Aminomethylpropanol | 0.5 |
| Isopentane | 6 |
| Water | to 100 |

The above composition was packed in a two-chamber aerosol can and pressurised with liquid nitrogen. The inner pressure was approximately 8.5 bar.

The above composition was released from the packaging as a clear gel and foamed subsequently within a seconds and was easily applied onto wet hair and hair was dried. It was observed in a half side test against a conventional gel composition that the above composition was performed better in temrs of easy application, gav hair more shine and hair treated had less flyaways.

Similar results were observed with the following examples.

### Example 2

| | % by weight |
|---|---|
| Propylene glycol | 5 |
| Glycerine | 5 |
| PVP | 2 |
| PEG-60 Hydrogenated castor oil | 2 |
| Synthalen W 2000 | 5 |
| Aminomethylpropanol | 0.5 |
| Basic red 51 | 0.2 |
| Isopentane | 6 |
| Water | to 100 |

The above composition was packed in a two-chamber aerosol can and pressurised with LPG to a pressure of around 8.5 bar.

The above composition additionally provided hair a red shiny shimmer.

### Example 3

| | % by weight |
|---|---|
| Propylene glycol | 5 |
| Panthenol | 5 |
| PVP | 2 |
| PEG-60 Hydrogenated castor oil | 2 |
| Synthalen W 2000 | 5 |
| Aminomethylpropanol | 0.5 |
| Benzophenone-4 | 0.1 |
| Basic red 51 | 0.1 |
| Basic orange 31 | 0.05 |
| Basic yellow 87 | 0.05 |
| Basic red 76 | 0.1 |
| Mint leaf extract | 0.5 |
| Isopentane | 6 |
| Water | to 100 |

The above composition was packed in a two-chamber aerosol can and pressurised with carbondioxide to a pressure of around 8.5 bar.

The above composition additionally provided hair a red - yellowish shiny shimmer.

## Claims

1. A post-foaming aqueous gel composition **characterized in that** it comprises one or more thickening polymer, one or more film forming polymer, one or more polyol, one or more surfactant and one or more C4 - C5 isoalkane.

2. The composition according to claim 1 **characterized in that** it comprises less than 5% by weight, calculated to the total composition, one or more surfactant.

3. The composition according to claims 1 and 2 **characterized in that** one or more thickening polymers are selected from the polymers forming a thixotropic gel in an aqueous medium.

4. The composition according to any of the preceding claims **characterized in that** one or more thickening polymer is an alkyl acrylate polymer and comprised at a concentration in the range of 0.1 to 5% by weight calculated to the total composition.

5. The composition according to any of the preceding claims **characterized in that** one or more surfactant is selected from nonionic surfactants, preferably from ethoxylated fatty alcohols and ethoxylated glycerides and their mixtures.

6. The composition according to any of the preceding claims **characterized in that** one or more polyol is selected from propylene glycol, glycerin and panthenol or their mixtures.

7. The composition according to any of the preceding claims **characterized in that** it comprises isopentane and/or isobutene and at a concentration in the range of 1 to 20% by weight calculated to the total composition.

8. The composition according to any of the preceding claims **characterized in that** one or more film forming polymer is selected from nonionic, cationic, anionic and amphoteric ones and included in the composition at a concentration in the range of 01 to 10% by weight calculated to the total composition.

9. The composition according to any of the preceding claims **characterized in that** it comprises one or more UV filter.

10. The composition according to any of the preceding claims **characterized in that** it comprises one or more hair direct dye.

11. A pressurized product for treating hair **characterized in that** it comprises a pressure resistant vessel consisting of two chambers wherein one of the chambers comprises the composition according to claims 1 to 10 and the other chamber comprises at least one pressurizing gas wherein the pressure inside the vessel does not exceed 12 bar.

12. The product according to claim 11 **characterized in that** a movable piston is present inside the pressurized vessel.

13. Use of the composition according to claims 1 to 10 for treating hair.

14. Process for treating hair **characterized in that** a composition according to claims 1 to 10 is applied onto hair, preferably onto wet hair.

15. Kit for hair comprising a composition according to claims 1 to 10.
